# EUROPEAN PATENT SPECIFICATION

(11) **EP 2 305 838 B1**
(45) Date of publication and mention of the grant of the patent: **28.01.2015**
(21) Application number: 09750794.1
(22) Date of filing: 21.05.2009
(51) Int. Cl.: C12N 1/20, A61K 35/74, A23C 9/127, C12R 1/225, A23L 1/30, C12P 17/18, A23L 1/314, A23C 19/00, A23G 3/00, A23C 9/12, A23C 9/13, C12R 1/01

(54) **BIFIDOBACTERIA THAT PRODUCES FOLIC ACID, FOOD COMPOSITION AND USE OF SAID BIFIDOBACTERIA**
BIFIDOBAKTERIEN, DIE FOLSÄURE PRODUZIEREN, NAHRUNGSMITTELZUSAMMENSETZUNG UND VERWENDUNG DER BIFIDOBAKTERIEN
BIFIDOBACTÉRIE PRODUCTRICE D'ACIDE FOLIQUE, COMPOSITION ALIMENTAIRE ET UTILISATION DE CETTE BIFIDOBACTÉRIE

(30) Priority: 21.05.2008 MX MX08006546
(43) Date of publication of application: 06.04.2011
(73) Proprietor: Sigma Alimentos, S.A. De C.V., Nuevo León (MX)
(72) Inventor: GARZA GONZÁLEZ, Elvira, Monterrey Nuevo León 67174 (MX); BOSQUES PADILLA, Francisco Javier, Monterrey Nuevo León 64630 (MX); NARANJO MODAD, Sandra, San Pedro Garza García Nuevo León 66250 (MX); MORENO CAMPAÑA, Víctor Manuel, Monterrey Nuevo León 64619 (MX)
(74) Representative: Elzaburu Marquez, Alberto
(86) International application number: PCT/MX2009/000046
(87) International publication number: WO 2009/142472

(56) References cited:
- EP-A1- 1 698 704
- WO-A1-2006/013588
- ES-T3- 2 290 762
- LIN M Y ET AL: "Folate levels in cultures of lactic acid bacteria", INTERNATIONAL DAIRY JOURNAL, vol. 10, no. 5-6, 2000, pages 409-413, XP002717177, ISSN: 0958-6946
- DEGUCHI Y ET AL: "COMPARATIVE STUDIES ON SYNTHESIS OF WATER-SOLUBLE VITAMINS AMONG HUMAN SPECIES OF BIFIDOBACTERIA", AGRICULTURAL AND BIOLOGICAL CHEMISTRY, JAPAN SOC. FOR BIOSCIENCE, BIOTECHNOLOGY AND AGROCHEM, TOKYO, JP, vol. 49, no. 1, 1 January 1985 (1985-01-01), pages 13-20, XP009040803, ISSN: 0002-1369
- D'AIMMO M R ET AL: "The potential of bifidobacteria as a source of natural folate", JOURNAL OF APPLIED MICROBIOLOGY, vol. 112, no. 5, May 2012 (2012-05), pages 975-984, XP002717178,
- LEBLANC J G ET AL: "Folate production by lactic acid bacteria and other food-grade microorganisms", COMMUNICATING CURRENT RESEARCH AND TRENDS IN APPLIED MICROBIOLOGY, FORMATEX, EXTREMADURA - SPAIN, vol. 1, 1 January 2007 (2007-01-01), pages 329-339, XP002547461, ISBN: 978-84-611-9422-3
- POMPEI A. ET AL.: 'Folate production by Bifidobacteria as a potential probiotic property' APPLIED AND ENVIRONMENTAL MICROBIOLOGY vol. 73, no. 1, January 2007, pages 179 - 185
- CRITTENDEN R. G. ET AL.: 'Synthesis and utilisation of folate by yoghurt starter cultures and probiotic bacteria' INTERNATIONAL JOURNAL OF FOOD MICROBIOLOGY vol. 80, 2003, pages 217 - 222
- POMPEI A. ET AL.: 'Administration of folate- producing bifidobacteria enhances folate status in Wistar rats' THE JOURNAL OF NUTRITION vol. 137, 2007, pages 2742 - 2746

## Description

### TECHNICAL FIELD OF THE INVENTION

The present invention relates, in general, to strains of bacteria belonging to the genus Bifidobacterium that are producing folic acid, and particularly to a pure culture of *Bifidobacterium animalis* strain producing folic acid, a food composition containing this strain and uses of the strain. The *Bifidobacterium animalis* is deposited with the American Type Culture Collection (ATCC) under the Budapest Treaty, dated on April 23, 2008, and respectively identified with the ATCC designation number for patent deposit PTA-9175.

### BACKGROUND OF THE INVENTION

Discovered in the 40's, folic acid is considered a water-soluble vitamin B complex. It is also known as folacine or folates whose etymology comes from the Latin *folium,* meaning leaf.

This vitamin is essential for carrying out all functions of our body. Its great importance is based on the fact that folic acid is essential at the cellular level to synthesize DNA (desoxyrribonucleic acid), which transmits the genetic characteristics, and also to synthesize RNA (ribonucleic acid) needed to form proteins, body tissues and other cell processes. Therefore, the presence of folic acid in the body is essential for the correct cell division and duplication.

The folates work together with vitamin B12 and vitamin C in the utilization of proteins. It is important to note that folic acid is essential for the formation of the hemo group (part of hemoglobin that contains iron), so it is related to the formation of red blood cells.

Folic acid also provides benefits to the cardiovascular and the nervous systems, and to the neurological fetal formation, among others. Given their great importance to humans, many of the foods we consume today contain additional folic acid.

This acid is formed in the intestine from the intestinal flora. It is mainly absorbed in the small intestine, then distributed in the tissues through the bloodstream and stored in the liver. It is excreted in the urine and feces.

Among the functions of folic acid we find that it acts as a coenzyme found in the process of transfer of carbon groups; interfers in the synthesis of purines and pyrimidines, thus participating in the metabolism of DNA, RNA and proteins; it is necessary for the formation of blood cells, more specifically of red blood cells; it reduces the risk of defect appearing in the neural tube of the fetus as there are the spina bifida and anencephalia; it diminishes the occurrence of cardiovascular diseases; it prevents some types of cancer; it helps to increase the appetite, and stimulates the formation of digestive acids.

Among the sources of folic acid there are the ones of animal origin, at very low levels such as in liver, chicken, milk and its derivatives; sources of vegetive origin such as vegetables (lentils, beans, soy), whole grains and their derivatives, green leaf vegetables (spinach, cabbage, lettuce, asparagus), wheat germs, and fruit (melon, bananas, planes, oranges and aguacate or avocado among others; and as supplements by means of compressed of folic acid.

With the handling of food, more than half the natural content of the folic acid may be lost or destroyed. It is destroyed with prolonged cooking in lots of water, by warming meals, and also by storing food at room temperature.

Folic acid deficiency can manifest itself through the following symptoms: megaloblastic anemia (immature red blood cells have a size larger than normal), low weight, poor appetite, weakness, paleness, fatigue, nausea, diarrhea, bad temper, depression, inflammation and sores of the tongue, mouth ulcers, tachycardia, slow growth and greying hair.

The best way to meet the daily requirement of this vitamin is through a balanced and equilibrated diet including all food groups. However, there are situations where folic acid supplements may be needed, such as for women of childbearing age, those who are pregnant or breastfeeding, in order to prevent defects in the neural tube of the fetus as there are the spina bifida and anencephalia; as it is considered that all women that take folic acid supplements before conception reduce by 50% the risks of neurological defects in the future child; in the case of older people, from the age of 65 the capacity of absorption of vitamins clearly diminishes; people who smoke due to the use of tabacco that obstructs the absorption and availability of complex B vitamins; in alcoholic persons as alcohol diminishes and hinders the absorption of vitamins; in persons with diseases with frequent evacuations and diarrhea that avoid good absorption of this vitamin; and in persons with frequent usage of certain medicines, such as oral contraceptives, anti-inflammatories, sedatives, sleeping pills, etc.

Table 1 sets out the recommended daily intake of folic acid according to the Nutrition Department of the IOM (Institute of Medicine) and USDA (United States Department of Agriculture) as well for infants, children as for adults.

**Table 1**

| Recommended daily intake of folic acid | | |
|---|---|---|
| Age | Men µg/day | Women µg/day |
| 1 to 3 years | 150 | |
| 4 to 8 years | 200 | |
| 9 to 13 years | 300 | |
| 14 to 18 years | 400 | |
| 19 years and older | 400 | |
| Pregnant | | 600 |
| Breastfeeding | | 500 |

Due to insufficient information regarding the recommended dosage of folate for infants, the adequate intake has been established based on the amount of folate consumed by healthy infants and who are fed through breast milk. This is 60 µg/day until 6 months and 80 µg/day until 12 months of age.

Added to this, it has been reported that a dosage of 400 µg/day of folic acid can decrease the levels of homocysteine concentration in the blood, thus reducing the risk of cardiovascular disease (Lynnette J. Riddell, Alexandra Chisholm, Sheila Williams, and Jim I. Mann, Dietary Determinants of Plasma Homocysteine Concentrations, Am J Clin Nutr 2000, 71:1448-54).

The risk of toxicity with folic acid intake from food as well as from supplements is low. Being a water soluble vitamin, any excess intake is excreted through urine. There is also evidence that some patients taking anticonvulsant drugs may experience seizures when taking high levels of folic acid.

As a reference, tolerable upper intake levels have been established (see Table 2) to prevent the risk of toxicity by the intake of folic acid. The adverse effects increase in relation to the higher intakes up to the maximum tolerable level. An intake greater than the maximum set can originate symptoms of vitamin B12 deficiency (nerve degeneration and masking of anemias) due to the interaction between them.

**Table 2**

| Maximum tolerable intake of folic acid | | |
|---|---|---|
| Age | Men µg/day | Women µg/day |
| 1 to 3 years | 300 | |
| 4 to 8 years | 400 | |
| 9 to 13 years | 600 | |
| 14 to 18 years | 800 | |
| 19 years and older | 1000 | |
| Pregnant | | 800-1000 |
| Breastfeeding | | 800-1000 |

It is thus of utmost importance to find means by which to provide the body with a natural endogenous source, non-toxic, capable of continuously providing the necessary amount of folic acid and thus provide an alternative to conventional methods of administration of said substance or its salts

The human digestive tract accommodates a plural number of bacteria living in symbiosis with the host. There are large differences in the microbial content between the different parts of the tract, appearing about 95% of all intestinal bacteria in the colon, which is the most important part of the intestine. It has been estimated that over 400 species of bacteria proliferate in the colon. Besides these, the bowel contains microbes microbes known as transient (GR Gibson and MB Roberfroid (eds.), Colonic Microbiota, Nutrition and Health, Kluwer Academic Publisher, Dordrecht, 1999). The dominant species are the following: *Bacteroides, Bifidobacterium, Coprococcus, Peptostretococcus, Eubacterium* and *Ruminococcus.* The number of species of *Lactobacillus, Streptococcus, Fusobacterium, Veillonella, Propionibacterium* and *Enterobacteriaceae* was slightly lower. Some of the species represent useful microbes, while others may even be harmful. The average microbial content of the feces is 1x10¹² cfu/g (per dry matter). The bacteria degrade and ferment those components of the food in the colon that are not absorbed in the small intestine, absorbing the end products of fermentation in the intestine for use by the body. Besides nutrition, microbial balance of the colon has a fundamental significance for the health of a man (Tannock, GW, 1998, Studies of the intestinal microflora: A prerequisite for the Development of probiotics, Int Dairy J., 8: 527-533). The changes in the composition of the intestinal flora or the sudden reduction of the amount of it (due to severe diarrhea, treatment with antibiotics, etc.) increase the infectivity of potentially pathogenic species, which can have serious consequences (risk of allergies, intestinal diseases, cancer).

The genus *Bifidobacterium* is highly known for its beneficial activity in the body. This activity is reflected, for example, in its ability to repopulate and compensate for the intestinal bacterial flora after an antibiotic therapy in maintaining a balance between the different intestinal microbial groups, in reducing cholesterol levels, in the production of vitamins, and relief from intolerance to lactose.

The bacteria belonging to the genus *Bifidobacterium* are considered probiotics that are commonly used in pharmaceutical, veterinary and/or food. Probiotics are live microbes that, when administered to humans or animals, promote the welfare of the host by improving intestinal microbial balance (Fuller, R. Probiotics in man and animals, 1989, J. Appl. Microbiol. 66: 365-378). The best-documented probiotics include L. Rhamnosus LGG, L. Johnsonii LAI, L. Casei Shirota and Bifidobacterium lactis Bbl2. Moreover, the technical literature has also described a number of other probiotics (see, for example, M.E. Sanders and J.H. in't Veld 1999, Antonie van Leeuwenhoek 76: 293-315, Kluwer Academic Publishers). The health-promoting effects of probiotics include the balance and maintenance of the intestinal flora, stimulation of the immune system, and anti-carcinogenic activity. Practical effects of probiotics in the human intestine are based on several factors caused by live bacterial cells, its cellular structures and its metabolic products. Probiotics are commonly administered in nutrients or as capsules.

A bacteria can be referred to as a probiotic, basically if it meets the following requirements (Lee, YK. And Salminen, S., 1995, The coming age of probiotics. Trend Food Sci. Technol., 6: 241-245): remains capable of living in the demanding conditions that prevail in the digestive tract (low pH gastric, acids in the digestive system, etc.), it adheres to the walls of the intestine, metabolizes in the intestine, is technologically applicable (endures the processed), shows effects on health that were clinically studied and described, and it may be safely consumed.

A current application of probiotic producer of folic acid pertaining to the genus *Bifidobacterium* is described by Giovanni Mogna and Gian Paolo Strozzi in the publication of the international patent application WO-2006/013588A1. The document describes bacterial strains of human origin that belong to the genus *Bifidobacterium* characterized for being producers of folic acid and used as probiotics in pharmaceutical, veterinarian and food formulas. In particular, the species described are *Bifidobacterium adolescentis, Bifidobacterium breve* and *Bifidobacterium pseudocatenulatum* placed in the center of the collection DSMZ (Deutsche Sammlung und Zellkulturen von Mikroorganismen GmbH, Braunsweig, Germany), according to the Budapest Treaty, on July 21, 2004.

Species of bifidobacteria described in this patent document WO-2006/013588A1 have a production level of folic acid according to the following: *Bifidobacterium adolescentis* identified as DMS 16594 of 56 ng/ml to 62 ng/ml; *Bifidobacterium adolescentis* identified as DMS 16595 of 16 ng/ml to 20 ng/ml; *Bifidobacterium breve* identified as DMS 16596 of 6 ng/ml to 9 ng/ml; *Bifidobacterium pseudocatenulatum* identified as DMS 16597 of 14 ng/ml to 16 ng/ml; and *Bifidobacterium pseudocatenulatum* identified as DMS 16598 of 14 ng/ml to 19 ng/ml. These levels of folic acid production are low compared with *Bifidobacteium animalis* PTA-9175 of this invention which is described below.

In addition to this, patent document WO-2006/013588A1 does not describe in detail nor identifies a species of *Bifidobacterium animalis* as a producer of folic acid at levels comparable to the production of folic acid of *Bifidobacterium animalis* PTA-9175 of the present invention, and moreover, can be used as a probiotic in food compositions.

R.G. Crittenden et al in "International Journal of food microbiology, 2003, vol.80, pages 217-222" examines different strains of bacteries used commonly in yogourts and fermented milks for their ability to synthetise and utilize folate during fermentation. Different strains of Bifidobacterium are tested more particularly Bifidobacterium animalis CSCC 1941 which produces about 23 ng/g of folic acid. It is shown also that a combination of S. thermophilus and B. animalis can increase the folic acid production to 70 ng/g.

Accordingly, there is a continuous and obvious need to offer consumers new products that have probiotic effects through the use of bifidobacteria which are capable of producing levels of folic acid in amounts much greater than the levels of folic acid production of bifidobacteria presently identified, the objective being to provide convenient products that are part of, complement of supplement of the daily diet of required folic acid.

### SUMMARY OF THE INVENTION

In view of the above mentioned and with the purpose of providing solutions to the limitations encountered, it is the object of the invention to provide a biologically pure culture of a strain of *Bifidobacterium animalis* PTA-9175 as a producer of folic acid.

Added to this, it is also the object of the invention to provide a food composition comprises a food product and a biologically pure culture of a strain of *Bifidobacterium animalis* PTA-9175 as a producer of folic acid.

It is also the object of the invention to provide a pharmaceutical composition for the treatment of a deficiency in folic acid that contains a biological pure culture of a strain of *Bifidobacterium animalis* PTA-9175 as a producer of folic acid.

Another object of the invention is a use of a strain of *Bifidobacterium animalis* PTA-9175 as probiotic producer of folic acid in food compositions.

Another object of the invention is the use of a strain of *Bifidobacterium animalis* PTA-9175 in pharmaceutical compositions for the treatment of the deficiency in folic acid.

Another object of the invention is to offer a method for producing a food product of fermented milk containing viable *Bifidobacterium animalis* and folic acid; the method consists of cultiving *Bifidobacterium animalis* PTA-9175 in a medium consisting of milk, reconstituted milk, whey, full milk or skimmed milk.

Another object of the invention is to offer a strain of *Bifidobacterium animalis* PTA-9175 as a producer of folic acid of from 120 ng/ml to 398 ng/ml.

### BRIEF DESCRIPTION OF THE FIGURES

The characteristic details of the invention are described in the following paragraphs together with the figures that can be found herein, which are for the purpose of defining the invention but without limiting its scope.

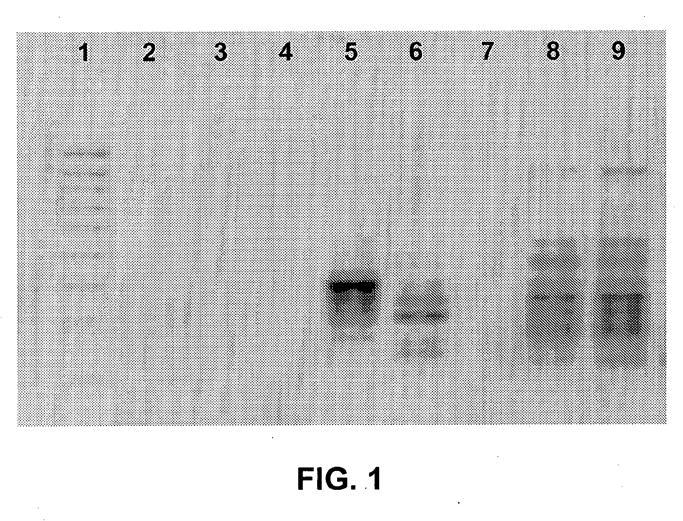

Figure 1 illustrates a molecular pattern of a strain of *Bifidobacterium animalis* PTA-9175 according to the invention, wherein lane 1 is the molecular weight marker of 100 bp and lanes 8 and 9 the *Bifidobacterium animalis* strain PTA-9175.

### DETAILED DESCRIPTION OF THE INVENTION

### IN VITRO TESTING

### Genus and species identification by a combination of phenotypic and genetic characteristics

### Phenotypic characteristics

Method: the API Rapid ID 32A system was used, which is a system of identification of anaerobic bacteria in 4 hours which includes enzymatic standardized tests, miniatunzed, and a specific database. The characterization was carried out according to the manufacturer's instructions. From a well isolated colony, a subculture was made in MRS agar and incubated for 48 hours in aneaerobiosis. A suspension of turbidity equal to 4 McFarland and inoculated with a gallery of 55 µl in each cupola. The cupola of URE, with 2 drops of paraffin oil and incubated for 4 hours at 37 °C in aerobiosis.

Results. The biochemical profile showed the following results contained in Table 3.

**Table 3**

| Reading of the results of phenotypic characterization of *Bifidobacterium animalis* strain PTA-9175 | |
|---|---|
| BIOCHEMICAL TEST | RESUTL |
| Urease production | Negative |
| Arginine dihydrolase | Negative |
| Alpha galactosidase | Positive |
| Beta-galactosidase 6-phosphate | Positive |
| Proline arylamidase | Negative |
| Alpha galactosidase | Positive |
| Beta galactosidase | Positive |
| Alpha arabinosidase | Positive |
| Beta galactosidase | Negative |
| Beta N-acetyl-glucosaminidase | Negative |
| Fermentation of mannose | Negative |
| Fermentation of rafinose | Positive |
| Ac. Glutamic decarboxylase | Negative |
| Alpha fucosidase | Negative |
| Nitrate reduction | Negative |
| Indole production | Negative |
| Alkaline phosphatase | Negative |
| Arginine arylamidase | Positive |
| Proline arylamidase | Positive |
| L-Leucyl glycine arylamidase | Positive |
| Phenylalanine arylamidase | Positive |
| Leucine arylamidase | Positive |
| Pyroglutamic acid arylamidase | Negative |
| Tyrosine arylamidase | Positive |
| Alanine arylamidase | Negative |
| Glycine arylamidase | Positive |
| Histidine arylamidase | Positive |
| Glutamyl glutamic acid arylamidase | Negative |
| Serine arylamidase | Positive |

According to the results of biochemical tests, which were performed at least 10 times, the strain was identified as *Bifidobacterium sp* with 99% certainty.

### Typing by sequencing of subunit 16S ribosomal

Method: the extraction of chromosomal DNA was performed from the *Bifidobacterium animalis* strain PTA-9175 using an extraction of phenol chloroform-isoamyl alcohol and ethanol precipitation.

A culture of *Bifidobacterium animalis* strain PTA-9175 was obtained after 48 hours; it was placed in a vial containing 700 µl of a solution of 100 mM Tris-HCL, 150 µg of lysozyme were added and it was incubated at 37 °C during 60 min in b.a.

After that time, 200 µl of TE1X (10 mM Tris-HCL and 1 mM EDTA, pH 8.0 with sodium dodecyl sulfate of 1% and 6 µl of proteinase K of 10 mg/ml concentration) were added and incubated at 55 °C during 60 °C in a water bath. The DNA was extracted by adding 500 µl of saturated phenol, 100 µl of SEVAG (chloroform-isoamyl alcohol), 200 µl of TE1X, mixed by inversion each time a reagent is added and a final 5 min agitation by inversion.

The sample was centrifuged at 14000 rpm during 8 minutes. From the aqueous phase the DNA was precipitated with 2.33 volumes of 98% ethanol at -20 °C during 24 hours. After the DNA was separated by centrifugation at 10000 rpm for 5 min and resuspended the DNA in 50 µl of TE1X.

It was left in cooling for 24 hours before quantified by fluorescence at 460 nm, and finally adjusted to a concentration of 100 ng/µl with TE1X.

The DNA concentration was determined by fluorometry and adjusted to 100 ng/ml.

### Amplification of the 16S ribosomal subunit

Initiators were used: lm26 F 5'gattctggctcaggatgaacg-3' and Im3 R 5'cgggtgcticccactttcatg-3'. The reaction mixture used was 25 µl, which contained, 2.5 µl of 10X buffer, 1.5 µl of MgCl₂ 50 mM, 17.25 µl of water, 0.25 µl of Taq polymerase, the final concentration of dNTP*'*s was 0.2 µM, each primer had a final concentration of 0.3 µl and 100 ng of DNA. The conditions of PCR amplification used were 35 cycles of denaturation at 94 °C for 60 seconds, aligning at 57 °C for 120 seconds, extension at 72 °C for 120 seconds, and finally a final extension cycle at 72 °C for 3 minutes; a thermocyclator equiment was used, model PX2 Thermal cycler (Thermo Electron Corporation, MA. U.S.A.).

The detection of amplified products was performed by electrophoresis in agarose gel at 1% ethidium bromide staining.

### Sequencing PTA-9175 Bifidobacterium animalis

| | |
|---|---|
| 1 | AATTAAAACC TTGGGYTTAC ATGCAGTCGA ACGGGATCCC TGGCAGCTTG CTGTCGGGGT |
| 61 | GAGAGTGGCG AACGGGTGAG TAATGCGTGA CCAACCTGCC CTGTGCACCG GAATAGCTCC |
| 121 | TGGAAACGGG TGGTAATACC GGATGCTCCG CTCCATCGCA TGGTGGGGTG GGAAATGCTT |
| 181 | TTGCGGCATG GGATGGGGTC GCGTCCTATC AGCTTGTTGG CGGGGTGATG GCCCACCAAG |
| 241 | GCGTTGACGG GTAGCCGGCC TGAGAGGGTG ACCGGCCACA TTGGGACTGA GATACGGCCC |
| 301 | AGACTCCTAC GGGAGGCAGC AGTGGGGAAT ATTGCACAAT GGGCGCAAGC CTGATGCAGC |
| 361 | GACGCCGCGT GCGGGATGGA GGCCTTCGGG TTGTAAACCG CTTTTGTTCA AGGGCAAGGC |
| 421 | ACGGTTTCGG CCGTGTTGAG TGGATTGTTC GAATAAGCAC CGGCTAACTA CGTGCCAGCA |
| 481 | GCCGCGGTAA TACGTAGGGT GCGAGCGTTA TCCGGATTTA TTGGGCGTAA AGGGCTCGTA |
| 541 | GGCGGTTCGT CGCGTCCGGT GTGAAAGTCC ATCGCCTAAC GGTGGATCTG CGCCGGGTAC |
| 601 | GGGCGGGCTG GAGTGCGGTA GGGGAGACTG GAATTCCCGG TGTAACGGTG GAATGTGTAG |
| 661 | ATATCGGGAA GAACACCAAT GGCGAAGGCA GGTCTCTGGG CCGTCACTGA CGCTGAGGAG |
| 721 | CGAAAGCGTG GGGAGCGAAC AGGATTAGAT ACCCTGGTAG TCCACGCCGT AAACGGTGGA |
| 781 | TGCTGGATGT GGGGCCCTTT CCACGGGTCC CGTGTCGGAG CCAACGCGTT AAGCATCCCG |
| 841 | CCTGGGGAGT ACGGCCGCAA GGCTAAAACT CAAAGAAATT GACGGGGGGC CCGCACAAGC |
| 901 | GGCGGAGCAT GCGGATTAAT TCGATGCAAC GCGAAGAACC TTACCTGGGC TTGACATGTG |
| 961 | CCGGATCGCC GTGGAGACAC GGTTTCCCTT CGGGGCCGGT TCACAGGTGG TGCATGCTCG |
| 1021 | TCGTCAGCTC GTGTCGTGAG ATGTGTGTTA AGTCCCGCAA CGAGCGCAAC CCTCGCCGCA |
| 1081 | TGTGCCAGGC GGGTGATGCC GGGAACTCAT GTTGGACCGT CGGGTCACTC GGAGGAGGTG |
| 1141 | GGGATGACGT CAGATCATCA TGCCCTTACG TCAGGCTCAC GCATGCTACA ATGCGCTACA |
| 1201 | CCCGGTGCGA CTGGTGACGT GGGGGCGATC GCTGAAAACC GGTCTCTCAG ATTTCGCGAT |
| 1261 | ACTGCAACAT CCTG |

### Sequence analysis

The sequence obtained was analyzed using the program Blast 2.2.14 and 99% homology was obtained with *Bifidobacterium animalis.* Table 4 shows the list of bacteria with the homology percentage of over 95%.

**Table 4**

| Organisms found with a similarity above 95%. Compared with samples from Gen Bank, in the *National Center for Biotechnology Information* | |
|---|---|
| Organism | Homology (%) |
| *Bifidobacterium animalis* | 99 |
| *Bifidobacterium animalis* | 99 |
| *Bifidobacrerium animalis* | 99 |
| *Bifidobacterium animalis* | 99 |
| *Bifidobacterium animalis* | 99 |
| *bifidobacteria lactis* | 99 |
| *Bifidobacterium animalis* | 99 |
| *B. lactis* | 99 |
| *Bifidobacterium sp.* | 99 |
| *Bifidobacterium animalis* | 98 |
| *Bifidobacterium infantis* | 98 |
| *Bifidobacterium pseudolongum* | 97 |
| *Bifidobacrerium pseudolongum* | 97 |
| *Bifidobacterium pseudolongum* | 98 |
| *Bacterium ic1332* | 96 |
| *Uncultured bacterium* | 96 |

### Typification of Bifidobacterium animalis strain PTA-9175 by RAPD

Method: the DNA extraction was carried out by the methodology described above for sequencing of the 16S ribosomal subunit.

The genotypification used the initiator: 5'- AAG TAA GTG ACT GGG GTG AGC G -3'. Reaction mixtures were prepared with a volume of 25 µl. Each sample contained the following: 200 µM of each desoxynucleoside triphosphate, 0.3 µM of initiator ERIC 2, 50 mM of KCI, 10 mM of Tris-HCl pH = 8.3, 3 mM of MgCl₂, 3 U of Taq DNA polymerase (Bioline, MA, USA), 500 ng of genomic DNA. The PCR conditions were as follows: initial denaturation of 1 cycle at 94 °C for 2 min, 40 cycles of denaturation at 94 °C for 20 sec, alignment at 35 °C for 20 sec and extension at 72 °C for 1 min; and finally a final extension of 1 cycle at 72 °C for 1 min using a thermocyclator Px2 Thermal Cycler (Termo Electron Corporation Milford, MA, U.S.A.). The amplified products were subject to electrophoresis in agarose gel at 2% stained with ethidium bromide were revealed in UV light. The pattern of amplifying bands was analyzed with the software Lab Works UVP Version 4.5 for Windows.

Results: A characteristic pattern was obtained of *Bifidobacterium animalis* strain PTA-9175. The pattern is shown in Figure 1.

### Determination of resistance patterns

Method: To determine the phenotype of resistance to antibiotics the Kirby-Bauer method was used. From a young culture of 18 to 24 hours several colonies were taken with a sterile swab and inoculated into a test tube with a sterile, isotonic saline solution. The inoculum was visually adjusted to a turbidity of 0.5 on the McFarland scale. Within 15 minutes the inoculum was adjusted, a sterile swab was introduced into the suspension and when removing it the excess liquid was eliminated. Agar Mueller-Hinton plates were inoculated, leaving no free zone, sliding the swab across the surface of the agar 3 times, rotating the plate 60° each time and finally passing it over the periphery of the agar to achieve a uniform seed.

The plates were left to dry for 3 to 5 min and the sensidiscs of the following antibiotics were placed: norfloxacin, cephalothin, azithromycin, tobramycin, ampicillin/sulbactam, erythromycin cefatoxim, nalidixic acid, ceftazidime, aztreonam, vancomycin, cefuroxime, rifampicyn, nitrofurantoin, oxacylin, nefilmicin amikacin, kanamycin ticarcillin, clindamycin, ampicillin.

Results: The results of sensibility testing are shown in Table 5. All strains with an inhibition diameter of 0 are resistant to the tested antibiotic. According to the results, it can be concluded that the strain of *Bifidobacterium animalis* PTA-9175 is resistant to aminoglycosides.

**Table 5**

| Results of the halos of inhibition of each of the tested antibiotics against the strain of *Bifidobacrerium animalis* PTA-9175 | |
|---|---|
| Antibiotic | Inhibition halo (mm) |
| Norfloxacin | 0 |
| Cephalothin | 24 |
| Azithromycin | 30 |
| Erythromycin | 40 |
| Ceftazidime | 38 |
| Vancomycin | 27 |
| Rifampicyn | 2 |
| Oxacylin | 0 |
| Nefilmicin | 0 |
| Kanamycin | 0 |
| Clindamycin | 43 |
| Tobramycin | 0 |
| Ampicillin/Sulbactam | 30 |
| Cefatoxim | 40 |
| Nalidixic acid | 0 |
| Aztreonam | 0 |
| Cefuroxime | 25 |
| Nitrofurantoin | 40 |
| Ofloxacin | 12 |
| Amikacin | 0 |
| Ticarcillin | 40 |
| Ampicillin | 35 |

### Determination of hemolysis in human blood

Method: MRS agar plates were prepared and supplemented with human blood at 5%. The plates were sown with *Bifidobacterium animalis* strain PTA-9175 and incubated under anaerobe conditions for 48 hours.

Result: No hemolysis was observed in the developed colonies of *Bifidobacterium animalis* strain PTA-9175.

### Resistance to stomach acid

Method: A bottle was prepared with 300 mL of HCl pH 1 and added 25 ml of milk pH 4.5 + 1 ml of a suspension of 10¹³ of *Bifidobacterium animalis* strain PTA-9175.

Gently mixed and sampled every 30 min until completing 3 hours. The samples were sown in an MRS medium and incubated under anaerobe conditions.

Results: Development was observed in all samples taken up to 3 hours. According to this result, it can be concluded that the strain of *Bifidobacterium animalis* PTA-9175 is resistant to the presence of acid.

### Bile resistance

Method: MRS agar plates were prepared and supplemented with human bile obtained by lapraoscopic cholecystectomy to final concentrations of 0.3%, 1%, 2%, 3%, 4%, 5%, 6% and 7.5%.

The plates were incubated in anaerobiosis at 37 °C for 72 hours. Moreover, a plate was sown from the same suspension of bacteria on a plate without bile.

We counted the number of colonies developed in the control plates and in the bile plates, and calculated the percentage of inhibition.

To determine whether the effect was bacteriostatic or bactericidal of the plates where no growth was found, a sample was taken from the agar surface with a swab to resow in an MRS medium without bile.

Results: On the control plate 115 colonies were developed, on plate 2 78 were developed, and on plate 3 17 colonies were developed. Thus the percentages of inhibition were 67.8% for the plate with 0.3% bile and 14.8% for the plate with 1% bile. There was no development on the plates with 2%, 3%, 4%, 5% and 6% bile.

Each of the plates where there was no development (from 4 to 8), were sown with MRS agar without bile to determine whether the effect was bacteriostatic or bactericidal. There was no growth on any plate, so it is assumed that a percentage of ≥2% bile has bactericidal effect on the *Bifidobacterium animalis* strain PTA-9175.

### Adherence to mucus and/or human epithelial cells and cell lines

We used the Caco-2 cell lines, HT-29.

Preparation of cell culture: To obtain the volume of cells needed to confront them with the *Bifidobacterium animalis* strain PTA-9175, the transfer technique was used or propagation of adherent cell lines forming a monolayer of a base culture to another one.

Starting from a vial with a stock of each of the cells, the complete modified Eagle medium (MEM) was inoculated with fetal calf serum to 20% and 50 µg of gentamicin per ml of complete medium for the Caco-2 and McCoy cells for cells HT-29. It was incubated at 37 °C with 5% of CO₂ and daily observed until observing a confluent growth.

The culture medium was removed, and added 1 ml trypsin 0.25% -EDTA, for each cm² of monolayer and allowed 1 hour of incubation at 37 °C until observed under the microscope that the cells were detached. The detached cells were collected in Falcon tubes and the trypsin was inactivated by adding 1.5 ml of complete medium for each ml of trypsin used.

Centrifuged at 1000 rpm for 3 min and the supernatant was removed with trypsin. The botton of obtained cells was washed with a solution of PBS 1X and again centrifuged under the same conditions.

The botton of cells was smoothly resuspended in 1 ml of MEM or MacCoy medium for each 10 _{CM}² of monolayer. From this suspension of cells aliquots were taken of 0.5 ml and transferred to bottles of 25 cm², and 7.5 ml of complete medium were added. Incubated at 37 °C in a humidified atmosphere of 5% CO₂ for 48 to 72 hours or until observing confluence in the bottles.

Confronting the *Bifidobacterium animalis* strain PTA-9175 to each of the cell lines: A bottle was prepared with a suspension of *Bifidobacterium animalis* strain PTA-9175 and 10⁵ bacteria were inoculated in the bottles of cell culture with a confluent culture. The incubation continued for 24 hours and observed under the microscope to find if the bacteria are attached to the cells by gentle movements of the bottle while under observation.

Results: Adhesion was observed of approximately 20% of the cells to the Caco-2 cell line. For cells HT-29 an adherence of approximately 30% was observed.

### Antimicrobial activity against pathogenies

Method: Young cultures were obtained from *S. aureus, E. coli O157:H7, Salmonella enteritidis, Campylobacter jejuni, Helicobacter pylori, Yersinia enterocolitica* and suspensions were prepared with turbidity corresponding to tube 1 of MacFarland.

The *Bifidobacterium animalis* strain PTA-9175 was cultivated in an MRS medium under anaerobic conditions at 37 °C for 48 hours. The suspension was made in a saline solution by adjusting the concentration to the corresponding for tube 4 of the MacFarland nephelometer.

The inoculum prepared was inoculated into an MRS agar of 3 mm depth per closed groove and with a swab in the center of each plate (an area of 1 by 1.5 inches). The plates were incubated in anaerobiosis at 37 °C for 48 hours. After that time, the plates were filled again with 10 ml of blood agar for *H. pylori* and with culture medium BHI for the rest of the bacteria. The medium was poured melted, sterilized and cooled to about 45 °C, previously inoculated with 1 ml of a suspension of the test pathogen. The agar was allowed to solidify and the plates were incubated at 37 °C in microaerobiosis for *H. pylori* and in aerobiosis for the rest of the bacteria, during 48 hours for *H. pylori* and 24 for the rest of the bacteria.

After observing the growth inhibition of the test pathogens, samples were taken from the zone of inhibition with a sterile swab and the corresponding culture medium was resown.

Result: Growth inhibition was detected in *S. aureus, E. coli O157:H7, Salmonella enteritidis, Campylobacter jejuni, Helicobacter pylori, Yersinia enterocolitica* through the strain of *bifidobacteria animalis* PTA-9175.

After observing the growth inhibition of the test pathogens, samples were taken from the zone of inhibition with a sterile swab and the corresponding culture medium was resown. Growth was observed in all the reseedings that were carried out, which showed that the effect of *Bifidobacterium animalis* strain PTA-9175 is bacteriostatic and not bactericidal.

### Activity of determination of extrachromosomic materials

Plasmids are extrachromosomal DNA elements that replicate intracellularly and independently, although in most cases they are not essential for the viability of the cell, they contain genes that confer different properties such as antibiotic resistance, virulence or metabolic activities.

For the investigation of plasmids from *Bifidobacterium animalis* strain PTA-9175, the extraction of plasmid DNA was carried out by alkaline lysis and visualization by agarose gel electrophoresis. Next, the procedure used is described.

Method: A young culture was resuspended in the strain of *Bifidobacteria animalis* PTA-9175 in 200 µL of lysis absorber: Solution I (glucose 50 mM, Tris-HCl 25 mM pH=8, EDTA 10 mM). After 5 min at room temperature, 400 µL of a freshly prepared alkaline solution were added (0.2 N NaOH, 1% SDS) and mixed by inversion from 3 to 6 times. The tube was placed in ice for 5 min, added 300 µL ammonium acetate 7.5 M pH = 7.8 and the tube content was mixed by gentle inversion for a few seconds. The tube was kept in ice for 10 min to allow the majority of proteins, the high molecular weight RNA and the chromosomal DNA to precipitate. Afterwards, it was centrifuged for 9 min at 14000 rpm and the supernatant was removed to a clean tube. 650 µL of isopropanol was added and incubated at room temperature for 20 to 30 min. Then, centrifuged at 14000 rpm for 15 min, the supernatant was decanted, the pellet was washed by inversion with 500 µL of 70% ethanol, centrifuged for 3 min at 14000 rpm and the inverted tubes were left on a sheet of paper for 14 min to dry. The pellet was dissolved in 50 to 100 µL of 1X TE, 5 µL RNA*'*sa of 1 mg / mL was added, and incubated for 15 min at 37 °C. A gel was run in agarose at 2% to detect the presence of genetic material.

Results: In the gel electrophoresis no genetic material was detected corresponding to plasmids.

### Bile hydrolase activity

Method: The hydrolase activity of bile salts was carried out by determining the release of amino acids of the bile salts.

A young culture was resuspended in the strain of *Bifidobacteria animalis* PTA-9175 in a phosphate absorber pH=7 and centrifuged at 10000 rpm at 4 °C. The cells were washed twice with a sodium phosphate absorber pH = 7 and resuspended in the same absorber to obtain a density of 5 to 10 optical units at 600 nm. Ten ml of this cell suspension was sonicated for 3 min and the mixture was centrifuged for 10 min at 20000 rpm. The supernatant was retained as a cell-free extract.

180 µl of aborber was mixed (0.1 M sodium phosphate pH=6) with a sample of 10 µl of a human bile salt mixture, and heated to 37 °C. A sample of 50 µl was taken after 10 and 30 min and the sample was immediately mixed with TCA at 15%. These samples were centrifuged at maximum speed to remove the precipitate.

For the second reaction, an aliquot of the supernatant was mixed with water to obtain a volume of 100 µl (20 of sample and 80 of water). To this mixture 1.9 ml of ninhydrin was added to a sodium citrate absorber of 0.5 M pH = 5.5, 1.2 ml of glycerol and 0.2 ml sodium citrate absorber pH = 5.5. This was strongly mixed and boiled for 14 min. Next, the tubes were cooled and the absorbance was measured at 570 nm.

Strains of *Lactobacillus, Enterococcus* and *Bacteroides* were used as controls of the production of bile hydrolase. The tests were performed in duplicate.

Results: Bile hydrolase activity was detected in the strains used as positive controls. Not any activity was detected in *Bifidobacterium animalis* strain PTA-9175.

Conclusions: The ability of probiotic bacteria to hydrolyze bile salts has been included among the criteria for selection of bacteria such as probiotics, and a wide range of bile salt hydrolases has been identified and characterized. However, the activity of this enzyme has also shown a harmful effect on the host and that some pathogenic bacteria such as *L. monocytogenes* and other potencially patogenes as *Enterococcus faecalis* produce it.

### IN VIVO TESTING

### Determination of the lack of infectivity in immunocompromised animals

Method and Results: We used BALB/c mice, male, purchased from Harlan-Mexico, 6 weeks of age.

A culture was prepared of 20 h of *Bifidobacterium animalis* strain PTA-9175 in milk medium (10% skim milk, cysteine hydrochloride at 0.05% and yeast extract at 0.5%). This culture was used to administer doses to the animals. The doses and frequency are detailed below.
Stage 1. Without cyclophosfamide
   Group 1. 10 mice
      Inoculum: 10¹⁰ twice daily for 18 days (0.2 ml/oral)
      Weight at the beginning of the treatment: 21.05 gr
      Weight at the end of the treatment: 19.58 gr
      1.47 gr of weight lost
   Group 2. 10 mice
      Inoculum: 10¹⁰ twice daily for 24 days (0.2 ml/oral)
      Weight at the beginning of the treatment: 21.74 gr
      Weight at the end of the treatment: 22.77 gr
      1.03 gr of weight gained
   Group 3. 10 mice
      Inoculum: 10¹⁰ twice daily for 18 days (0.2 ml/oral)
      Weight at the beginning of the treatment: 22.32 gr
      Weight at the end of the treatment: 21.09 gr
      1.23 gr of weight lost
   Group 4. 10 mice
      Inoculum: 10¹⁰ twice daily for 24 days (0.2 ml/oral)
      Weight at the beginning of the treatment: 23.09 gr
      Weight at the end of the treatment: 23.8 gr
      They won: 0.71 gr of weight
Stage II. With cyclophosfamide (200 mg/kg every 7 days)/IP (permanent neutropenia)
   Group 1. 10 mice
      Inoculum: 10¹⁰ twice daily for 18 days (0.2 ml/oral)
      Weight at the beginning of the treatment: 22.23 gr
      Weight at the end of the treatment: 19.06 gr
      Lost: 3.17 gr of weight
   Group 2. 10 mice
      Inoculum: 10¹⁰ twice daily for 24 days (0.2 ml/oral)
      Weight at the beginning of the treatment: 21.88 gr
      Weight at the end of the treatment: 19.68 gr
      Lost: 2.20 gr of weight
   Group 3. 10 mice
      Inoculum: 10¹⁰ twice daily for 18 days (0.2 ml/oral)
      Weight at the beginning of the treatment: 20.84 gr
      Weight at the end of the treatment: 17.17 gr
      Lost: 3.67 gr of weight
   Group 4. 10 mice
      Inoculum: 10¹⁰ twice daily for 24 days (0.2 ml/oral)
      Weight at the beginning of the treatment: 20.97 gr
      Weight at the end of the treatment: 19.75 gr
      Lost: 1.22 gr of weight

Handling of animals at the end of the periods as indicated. At the end of the periods indicated, the animals were sacrificed by cervical dislocation. Extracted esophagus, stomach, cecum, kidney, spleen, liver. These were placed in jars with formaldehyde and sent for anatomopatologic study.

Observations in the animals during the study: During the study, the mice from the group without cyclophosphamide had no fever, no change in behavior, or changes in fur. During the study, the mice from the group with cyclophosphamide had no fever, nor did they present any changes in fur, and their behavior was to remain in group for long periods.

In the organs removed no macroscopic changes were observed in color nor in size. There was no abscess formation.

In the histological study no presence of bacteria was found in any of the samples. No histological changes were seen with inflammation. Alterations were detected stress-related in liver and spleen.

Conclusions: Under the conditions of this study, oral administration in normal mice and mice treated with cyclophosphamide with *Bifidobacterium animalis* strain PTA-9175 showed no visible toxicity.

### Determination of adverse effects during studies on humans

Selection of individuals: We assessed 24 healthy individuals who underwent single stool parasite search and complete examination to confirm their good health including blood sampling for conducting baseline blood chemistry (glucose, urea and creatinine), a complete hematic biometry, and a pregnancy test for the women.

All individuals of the study showed normal values in blood chemistry parameters and in hematologic biometry.

The individuals were divided randomly into two groups to receive a placebo (group A) (n=13, mean age = 25, range = 19-50, F/M = 7/6) or the yogurt supplemented with the *Bifidobacterium animalis* strain PTA-9175 (group B) (n=11, mean age = 24, range = 19-33, F/M=8/3).

Design phase: This phase lasted two weeks, during which they were given two bottles of yogurt daily of 250 mL each according to the test group.

Clinical assessment: The individuals were instructed to refer any change in bowel habits or intake of any medication that could interfere with the study.

Collection of stool samples: After one to two weeks of the ingestion of yogurt stool samples were collected to search for Bifidobacteria and its molecular typification.

### Analysis of Bifidobacteria

The first and second week of study, 3 colonies were identified as Bifidobacteria of each patient and each was grown for biomass collection, DNA extraction and molecular identification using a PCR.

All strains were subjected to genotypification using the primer 5'-AAG TAA GTG ACT GGG GTG AGC G-3' for identification of the characteristic pattern.

To complement the study, the cultivation, DNA extraction and genotypification by ERIC-PCR were carried out in the strain of *Bifidobacterium animalis* PTA-9175 that was administered.

The study timetable is shown in Table 6.

**Table 6**

| Study timetable | | | |
|---|---|---|---|
| Procedure | Week of Study | | |
| | 0 | 1 | 2 |
| Clinical assessment | X | X | x |
| Ingestion of yogurt | | X | X |
| Stool culture for Bifidobacteria | | X | X |

### Results

Leukocyte count: No differences were observed in the counting of the leukocytes between the sample of baseline blood and the sampling of the first study week in none of the two study groups (p<0.05).

Weight: No increase nor decrease in weight was detected during the two weeks of study in any of the two groups (p<0.05).

Blood Pressure: No increase nor decrease in the blood pressure weight was detected during the two weeks of study in any of the groups.

Bristol Scale. In group A, 4 patients increased to a value on the scale of Bristol and the rest remained unchanged.

In group B, 1 patient increased one value on the scale of Bristol. The rest remained unchanged.

Stool color: No changes in stool color in any of the study groups.

Stool consistency: 5 patients in group A improved stool consistency, and in group B 5 patients improved stool consistency.

There was no difference between the study groups (p<0.05).

Number of stools: There was no difference in the frequency of stools between the study groups (p<0.05). (p<0.05).

Number of stools: There was no difference in the frequency of stools between the study groups (p<0.05). (p<0.05).

Satisfaction after the first and second week of intake of yogurt: In group A, two subjects reported feeling very well after two weeks of consumption of yogurt. Two individuals mentioned they felt very well.

In group B, one person mentioned he felt regular the first week, and in the second week one of them said to feel fine. All other reports mentioned that people felt well.

Constipation: One patient in group A and one patient in group B reported constipation in the first week of consumption of the yogurt. Constipation got resolved by the second week of eating yogurt.

Diarrhea: None of the individuals in the study described the presence of diarrhea.

Abdominal pain: In group A, no individual reported abdominal pain. In group B, one person reported a mild abdominal pain which persisted during the second week.

Postprandial satiety: In group A there were no reports of postprandial satiety. In group B, two patients reported postprandial satiety in the first week, which disappeared the second week of consumption of the yogurt.

Flatulence: There was no difference in the presence of flatulence between the study groups.

Abdominal distension: None of the individuals in Group A showed any abdominal distension. One of the persons in group B showed abdominal distension in the first week of the product consumption, which disappeared the second week of consumption of the yogurt.

Adverse effects: Two patients in group A developed fever during the study. Neither needed antibiotics, and therefore they continued in the study.

One of the patients had a clinically documented viral pharyngitis. The other patient had a febricula that developed with myalgia and arthralgia. The discomfort was gone in about 6 hours.

Analysis of Bifidobacteria: We carried out a genotypification of all strains of Bifidobacteria recovered and found a characteristic pattern of *Bifidobacterium animalis* strain PTA-9175 in 5 persons from Group B and in none of the persons from group A.

Conclusion: In the human studies no data were found to suggest infectivity of *Bifidobacterium animalis* strain PTA-9175.

### CHARACTERISTICS OF THE STRAIN OF BIFIDOBACTERIUM ANIMALIS PTA-9175

From the above results, we conclude that the strain of *Bifidobacterium animalis* PTA-9175 of the invention has the desirable characteristics of a probiotic strain, as:
- It corresponds to the species *Bifidobacterium animalis,* which has proven probiotic activity in several publications.
- It is of human origin, particularly from the feces of infants.
- It is not hemolytic.
- Its morphology is of a Gram-positive bacterium in the form of a non-porulated bacillus.
- Its level of folic acid production is from 120 ng/ml to 398 ng/ml.
- It resists the presence of bile, even in small quantities.
- It is resistant to gastric pH.
- It is able to attach to intestinal cells.
- It inhibits the *in vitro* development of various pathogenic bacteria.
- It demonstrated safety when administered in immunosuppressed animals.
- It showed that it is safe for administering to humans.
- It has no plasmids.

### FOOD PREPARATION CONTAINING BIFIDOBACTERIUM ANIMALIS STRAIN PTA-9175

Below are examples of achievements for food preparation containing *Bifidobacterium animalis* strain PTA-9175.

A preparation method for producing fermented milk food products, for example, yogurt containing *Bifidobacterium animalis* strain PTA-9175, is to add for its cultivation at least 25% of culture of *Bifidobacterium animalis* strain PTA-9175 to the total volume of full milk, reconstituted milk, whey or skimmed milk and mix them; then later add the fruit flavor, water, sweeteners, flavorers or fruit pulp requested, in order to produce a new milk drink, and shake it. The amount of culture of *Bifidobacterium animalis* strain PTA-9175 added may vary and could be added in greater or lesser amount. All the ingredients to grow a strain of *Bifidobacterium animalis* PTA-9175 are of a food grade, so that the culture is 100% safe and can be consumed directly after you have added the desired taste.

In foods such as cheese, the culture of *Bifidobacterium animalis* strain PTA-9175 can be added in the last phase of the product elaboration. In other products such as powdered milk, juices, coffee, soft drinks, powders for drinks, desserts, processed meats, sweets, etc., The culture of *Bifidobacterium animalis* strain PTA-9175 can be liodilzated and after once obtained the powder it van added to sais products in the final stage, where it should not be submitted to a later process of heating. In the case of frozen and prepared foods, the culture may be added once the product is ready for consumption. It is important not to add the culture of *Bifidobacterium animalis* strain PTA-9175 if the food will be subjected to heating.

In an alternative embodiment the food product may also include other probiotic bacteria related to *Bifidobacterium animalis* strain PTA-9175.

In another embodiment, the *Bifidobacterium animalis* strain PTA-9175 can be used to prepare a pharmaceutical product for the treatment of the deficiency in folic acid.

Based on the embodiments described above, it is contemplated that modifications of the embodiments described, as well as alternative embodiments will be considered obvious to a person skilled in the art of the art under this description. It is therefore considered that the claims cover said modifications and alternatives that are within the scope of this invention or their equivalents.

## Claims

1. A biologically pure culture of *Bifidobacterium animalis* strain PTA-9175 as a producer of folic acid.

2. The biologically pure culture according to claim 1, **characterized in that** the folic acid production by *Bifidobacterium animalis* strain PTA-9175 is from 120 ng/ml to 398 ng/ml.

3. A food composition comprising:
a food product; and
a biologically pure culture of *Bifidobacterium animalis* strain PTA-9175 as a producer of folic acid.

4. The food composition according to claim 3, **characterized in that** said food product is selected from a group consisting of fermented milk products, milk powder, cheese, cold meats, soft drinks, beverage powders, juices, sweets and combinations thereof.

5. The food composition according to claim 3, **characterized in that** the folic acid production by *Bifidobacterium animalis* strain PTA-9175 is from 120 ng/ml to 398 ng/ml.

6. The food composition according to claim 3, **characterized in that** further includes probiotic bacteria related to the *Bifidobacterium animalis* strain PTA-9175.

7. A pharmaceutical composition for the treatment of a deficiency in folic acid comprising a biological pure culture of a strain of *Bifidobacterium animalis* PTA-9175 as a producer of folic acid.

8. The pharmaceutical composition according to claim 7, **characterized in that** the folic acid production by *Bifidobacterium animalis* strain PTA-9175 is 120 ng/ml to 398 ng/ml.

9. Use of a strain of *Bifidobacterium animalis* PTA-9175 as a probiotic producer of folic acid in food compositions.

10. The use according to claim 9, **characterized in that** the folic acid production by *Bifidobacterium animalis* strain PTA-9175 is from 120 ng/ml to 398 ng/ml.

11. The use according to claim 9, for the preparation of fermented milk food.

12. Use of a strain of *Bifidobacterium animalis* PTA-9175 in pharmaceutical compositions for the treatment of the deficiency in folic acid.

13. The use according to claim 12, **characterized in that** the folic acid production by *Bifidobacterium animalis* strain PTA-9175 is from 120 ng/ml to 398 ng/ml.

14. A method for producing a food product of fermented milk containing viable *Bifidobacterium animalis* and folic acid; the method comprises the step of cultivating *Bifidobacterium animalis* PTA-9175 in a medium consisting of milk, reconstituted milk, whey, full milk or skimmed milk.

15. The method according to claim 14, **characterized in that** the folic acid production by *Bifidobacterium animalis* strain PTA-9175 is from 120 ng/ml to 398 ng/ml.

## Patentansprüche

1. Biologisch reine Kultur des *Bifidobacterium animalis*-Stamms PTA-9175 als Erzeuger von Folsäure.

2. Biologisch reine Kultur nach Anspruch 1, **dadurch gekennzeichnet, dass** die Erzeugung von Folsäure durch den *Bifidobacterium animalis-Stamm* PTA-9175 zwischen 120 ng/ml und 398 ng/ml liegt.

3. Nahrungsmittelzusammensetzung, Folgendes umfassend:
ein Nahrungsmittelprodukt; und
eine biologisch reine Kultur des *Bifidobacterium animalis-Stamms* PTA-9175 als Erzeuger von Folsäure.

4. Nahrungsmittelzusammensetzung nach Anspruch 3, **dadurch gekennzeichnet, dass** das Nahrungsmittelprodukt aus einer Gruppe ausgewählt wird bestehend aus fermentierten Milchprodukten, Milchpulver, Käse, Aufschnitt, Erfrischungsgetränken, Getränkepulver, Säften, Süßigkeiten und Kombinationen aus diesen.

5. Nahrungsmittelzusammensetzung nach Anspruch 3, **dadurch gekennzeichnet, dass** die Erzeugung von Folsäure durch den *Bifidobacterium animalis-Stamm* PTA-9175 zwischen 120 ng/ml und 398 ng/ml liegt.

6. Nahrungsmittelzusammensetzung nach Anspruch 3, **dadurch gekennzeichnet, dass** sie weitere probiotische Bakterien enthält, die mit dem *Bifidobacterium animalis-Stamm* PTA-9175 verwandt sind.

7. Pharmazeutische Zusammensetzung zur Behandlung eines Folsäuremangels umfassend eine biologisch reine Kultur eines Stamms von *Bifidobacterium animalis* PTA-9175 als Erzeuger von Folsäure.

8. Pharmazeutische Zusammensetzung nach Anspruch 7, **dadurch gekennzeichnet, dass** die Erzeugung von Folsäure durch den *Bifidobacterium animalis-Stamm* PTA-9175 zwischen 120 ng/ml und 398 ng/ml liegt.

9. Verwendung eines Stammes von *Bifidobacterium animalis* PTA-9175 als probiotischem Erzeuger von Folsäure in Nahrungsmittelzusammensetzungen.

10. Verwendung nach Anspruch 9, **dadurch gekennzeichnet, dass** die Erzeugung von Folsäure durch den *Bifidobacterium animalis-Stamm* PTA-9175 zwischen 120 ng/ml und 398 ng/ml liegt.

11. Verwendung nach Anspruch 9, zur Herstellung von fermentierten Milchprodukten.

12. Verwendung eines Stammes von *Bifidobacterium animalis* PTA-9175 in pharmazeutischen Zusammensetzungen zur Behandlung des Folsäuremangels.

13. Verwendung nach Anspruch 12, **dadurch gekennzeichnet, dass** die Erzeugung von Folsäure durch den *Bifidobacterium animalis-Stamm* PTA-9175 zwischen 120 ng/ml und 398 ng/ml liegt.

14. Verfahren zum Herstellen eines Nahrungsmittelprodukts aus fermentierter Milch mit lebensfähigem *Bifidobacterium animalis* und Folsäure; wobei das Verfahren den Schritt des Kultivierens von *Bifidobacterium animalis* PTA-9175 in einem Medium bestehend aus Milk, rekonstituierter Milch, Molke, Vollmilch oder entrahmter Milch umfasst.

15. Verfahren nach Anspruch 14, **dadurch gekennzeichnet, dass** die Erzeugung von Folsäure durch den *Bifidobacterium animalis-Stamm* PTA-9175 zwischen 120 ng/ml und 398 ng/ml liegt.

## Revendications

1. Culture biologiquement pure de la souche *Bifidobacterium animalis* PTA-9175 comme producteur d'acide folique.

2. Culture biologiquement pure selon la revendication 1, **caractérisée en ce que** la production d'acide folique par la souche *Bifidobacterium animalis* PTA-9175 est de 120 ng/ml à 398 ng/ml.

3. Composition alimentaire comprenant :
un produit alimentaire ; et
une culture biologiquement pure de la souche *Bifidobacterium animalis* PTA-9175 comme producteur d'acide folique.

4. Composition alimentaire selon la revendication 3, **caractérisée en ce que** ledit produit alimentaire est choisi dans un groupe constitué de produits laitiers fermentés, de poudre de lait, de fromage, de viandes froides, de sodas, de boissons en poudres, de jus, de friandises et de leurs combinaisons.

5. Composition alimentaire selon la revendication 3, **caractérisée en ce que** la production d'acide folique par la souche *Bifidobacterium animalis* PTA-9175 est de 120 ng/ml à 398 ng/ml.

6. Composition alimentaire selon la revendication 3, **caractérisée en ce qu'**elle comprend en outre des bactéries probiotiques apparentées à la souche *Bifidobacterium animalis* PTA-9175.

7. Composition pharmaceutique pour le traitement d'une carence en acide folique comprenant une culture biologique pure d'une souche de *Bifidobacterium animalis* PTA-9175 comme producteur d'acide folique.

8. Composition pharmaceutique selon la revendication 7, **caractérisée en ce que** la production d'acide folique par la souche *Bifidobacterium animalis* PTA-9175 est de 120 ng/ml à 398 ng/ml.

9. Utilisation d'une souche de *Bifidobacterium animalis* PTA-9175 comme producteur probiotique d'acide folique dans des compositions alimentaires.

10. Utilisation selon la revendication 9, **caractérisée en ce que** la production d'acide folique par la souche *Bifidobacterium animalis* PTA-9175 est de 120 ng/ml à 398 ng/ml.

11. Utilisation selon la revendication 9 pour la préparation d'un aliment laitier fermenté.

12. Utilisation d'une souche *Bifidobacterium animalis* PTA-9175 dans des compositions pharmaceutiques pour le traitement de la carence en acide folique.

13. Utilisation selon la revendication 12, **caractérisée en ce que** la production d'acide folique par la souche *Bifidobacterium animalis* PTA-9175 est de 120 ng/ml à 398 ng/ml.

14. Procédé de production d'un produit alimentaire de lait fermenté contenant une souche *Bifidobacterium animalis* viable et de l'acide folique ; le procédé comprenant l'étape de culture de la souche *Bifidobacterium animalis* PTA-9175 dans un milieu constitué de lait, de lait reconstitué, de petit lait, de lait entier ou de lait écrémé.

15. Procédé selon la revendication 14, **caractérisé en ce que** la production d'acide folique par la souche *Bifidobacterium animalis* PTA-9175 est de 120 ng/ml à 398 ng/ml.
